# EUROPEAN PATENT APPLICATION

(11) **EP 4 609 848 A1**
(43) Date of publication of application: **03.09.2025**
(21) Application number: 24382231.9
(22) Date of filing: 01.03.2024
(51) Int. Cl.: A61K 8/34, A61K 8/73, A61K 8/92, A61K 8/9789, A61Q 19/08

(54) **METHOD FOR OBTAINING A COMPOSITION COMPRISING CRUDE WATER EXTRACT OF GALEGA OFFICINALIS L. WATER, HIPPOPHAE RHAMNOIDES OIL AND RESVERATROL**

(71) Applicant: I-D Actives, S.L., 11403 Jerez de la Frontera Cádiz (ES)
(72) Inventor: CARBAJO ESPEJO, José Manuel, 11403 Jerez de la Frontera (ES); ASÍN LLORCA, Manuel, 11403 Jerez de la Frontera (ES)
(74) Representative: Hoffmann Eitle

(57) **Abstract**

The present invention refers to the cosmetic and medical field. Particularly, the present invention refers to methods for obtaining a composition comprising crude water extract of *Galega officinalis L.* water, *Hippophae rhamnoides* oil and resveratrol. It also refers to anti-ageing compositions obtainable by any of these methods, and to the same compositions for use as a medicament. It also refers to the cosmetic use of these compositions for skin care or maintenance, preferably for decreasing a sign of cutaneous aging, and to a cosmetic method for skin care or maintenance, comprising applying or administering to a subject a preparation for skin care or maintenance, wherein the preparation comprises the cosmetic composition of the invention.

## Description

### FIELD OF THE INVENTION

The present invention refers to the cosmetic and medical field. Particularly, the present invention refers to methods for obtaining a composition comprising crude water extract of *Galega officinalis L.* water, *Hippophae rhamnoides* oil and resveratrol. It also refers to anti-ageing compositions obtainable by any of these methods, and to the same compositions for use as a medicament. It also refers to the cosmetic use of these compositions for skin care or maintenance, preferably for decreasing a sign of cutaneous aging, and to a cosmetic method for skin care or maintenance, comprising applying or administering to a subject a preparation for skin care or maintenance, wherein the preparation comprises the cosmetic composition of the invention.

### STATE OF THE ART

In recent years, there has been an increasing demand for cosmetic and therapeutic compositions for skin care or maintenance. Particularly, there is a demand for compositions with anti-ageing properties, or compositions that decrease signs of skin aging. The present invention is focused on solving this problem and a method for obtaining a composition with improved anti-ageing properties, as well as cosmetic and therapeutic compositions for skin care or maintenance are herein provided.

### DESCRIPTION OF THE INVENTION

### Brief description of the invention

As explained above, the present invention refers to methods for obtaining a composition comprising crude water extract of *Galega officinalis L.* water, *Hippophae rhamnoides* oil and resveratrol. It also refers to anti-ageing compositions obtainable by any of these methods, and to the same compositions for use as a medicament. It also refers to the cosmetic use of these compositions for skin care or maintenance, preferably for decreasing a sign of cutaneous aging, and to a cosmetic method for skin care or maintenance, comprising applying or administering to a subject a preparation for skin care or maintenance, wherein the preparation comprises the cosmetic composition of the invention.

The inventors of the present invention hypothesised that the combination of galega water, sea buckthorn oil and resveratrol could be useful for the generation of a composition with improved anti-ageing properties.

However, when creating a composition comprising these three elements, they faced a major challenge: the aqueous nature of galega water did not allow the dissolution of the oil or the resveratrol due to its hydrophobic character, so that an alternative must be considered in order to solubilize the oil. As a potential solution, the inventors thought of using cyclodextrins (CDs), which are cyclic oligosaccharides composed of 6, 7, 8 or more glucopyranose units linked by α-(1, 4) bonds, forming a conical structure with a hydrophobic internal cavity that can form inclusion complexes with a wide variety of molecules through non-covalent interactions. These molecules have the peculiarity of having a hydrophobic core and a hydrophilic external part. These characteristic makes them capable of encapsulating molecules containing a hydrophobic fraction and a suitable size to enter the CD cavity.

The inventors also realised that, since resveratrol is sensitive to sunlight, high temperatures and certain pH conditions, encapsulating it with CDs could be useful not only to improve its solubility in water, but also protect resveratrol molecules thereby maintaining their antioxidant capacity and beneficial effects.

The inventors of the present invention successfully developed a method for the obtention of a composition comprising crude water extract of *Galega officinalis L.* water, *Hippophae rhamnoides* oil and resveratrol (**Example 2.1**). In addition, *in vitro* studies indicate that the composition obtainable by this method displays anti-ageing properties that makes it suitable for cosmetic and/or medical uses (**Example 2.2, 2.3, 2.4 and 2.5**). Importantly, this composition unexpectedly displays an increased antioxidant activity compared to crude water extract of *Galega officinalis L.* water, *Hippophae rhamnoides* oil and resveratrol alone (**Table 2**).

The first embodiment of the invention refers to a method (hereinafter referred to as the method of the invention) for obtaining a composition comprising crude water extract of *Galega officinalis L.* water, *Hippophae rhamnoides* oil and resveratrol, comprising the steps of:
a. solubilizing *Hippophae rhamnoides* oil in crude water extract of *Galega officinalis L.* through its molecular encapsulation with complexing agents,
b. solubilizing resveratrol in crude water extract of *Galega officinalis L.* through its molecular encapsulation with complexing agents, and
c. mixing both preparations and filtering the mixture.

In a preferred embodiment, the method is characterized in that the complexing agent of step a) is β-cyclodextrin, or in that the complexing agent of step b) is 2-hydroxypropyl-β-cyclodextrin.

In a preferred embodiment, the method is characterized in that the complexing agent of step a) is β-cyclodextrin, and in that the complexing agent of step b) is 2-hydroxypropyl-β-cyclodextrin.

The second embodiment of the invention refers to an anti-ageing composition (hereinafter referred to as the anti-ageing composition of the invention) obtainable by any of the methods of the invention.

In a preferred embodiment, the anti-ageing composition is characterized in that the *Hippophae rhamnoides* oil is extracted from the pulp and/or from the seed of the plant.

In a preferred embodiment, the crude water extract of *Galega officinalis L.* comprises biguanides.

In a preferred embodiment, the anti-ageing composition is characterized in that the resveratrol is at a final concentration of 10.0 mg/mL to 1.0 mg/mL, preferably at a final concentration of 2.5 mg/mL.

In a preferred embodiment, the anti-ageing composition is characterized in that the *Hippophae rhamnoides* oil is at a final concentration of 25 µL/mL to 1 µL/mL, preferably at a final concentration of 8 µL/mL.

The third embodiment of the invention refers to any of the anti-ageing compositions of the invention for use as a medicament.

In a preferred embodiment the anti-ageing compositions of the invention are for use in a method for the treatment of skin aging, the prevention of skin ageing, the promotion of skin redensification, the reduction of fibroblast senescence, the reduction of metalloproteinase function, the promotion of skin regeneration and/or the promotion of cutaneous healing.

The fourth embodiment of the invention refers to the cosmetic use of any of the anti-ageing compositions of the invention for skin care or maintenance, preferably for decreasing a sign of cutaneous aging, for renovating the skin, for regenerating the skin, for preventing or treating skin desquamation, for skin depigmentation, for reducing skin inflammation and/or for promoting cutaneous healing, more preferably for decreasing a sign of cutaneous aging.

The fifth embodiment of the invention refers to a cosmetic method for skin care or maintenance, comprising applying or administering to a subject a preparation for skin care or maintenance, wherein the preparation comprises the composition of any one of claims 4 to 8
In a preferred embodiment the cosmetic the subject is interested in achieving a decrease in a sign of skin aging.

In a preferred embodiment the composition is administered topically.

In a preferred embodiment the method comprises obtaining a composition comprising crude water extract of *Galega officinalis L.* water, *Hippophae rhamnoides* oil and resveratrol using the any of the methods of claims 1 or 2.

**In the context of the present invention the following terms are defined:**
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of'. Thus, the phrase "consisting of" indicates that the listed elements are required or mandatory, and that no other elements may be present.
- The term "cosmetically acceptable excipients or carriers" refers to excipients that may optionally be included with the cosmetic composition of the invention and that causes no toxic effects.

### Description of the figures

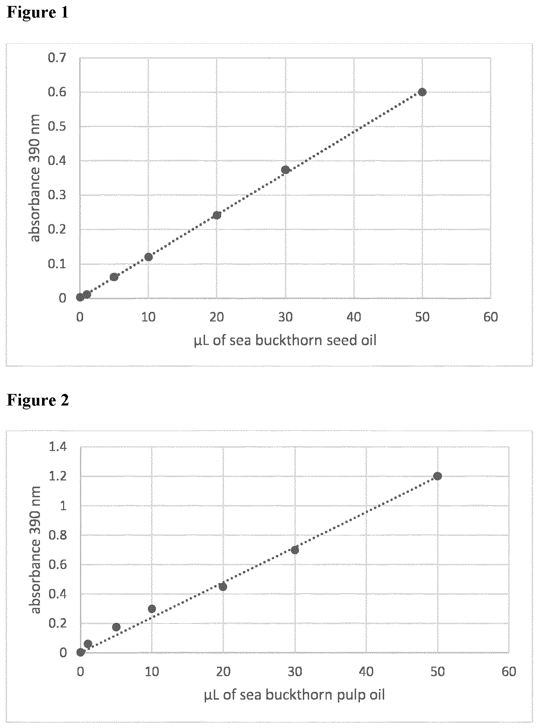

**Figure 1****.** Calibration line of sea buckthorn oil is extracted from the seed of the plant.
**Figure 2****.** Calibration line for sea buckthorn oil extracted from the pulp of the plant.
**Figure 3****.** Phase diagram of sea buckthorn pulp oil with β-CDs (grey) and HP-β-CDs (black).
**Figure 4****.** Phase diagram of sea buckthorn seed oil with β-CDs (grey) and HP-β-CDs (black).
**Figure 5****.** Phase diagram of resveratrol with β-CDs (grey) and HP-β-CDs (black).
**Figure 6****.** Phase diagram of resveratrol with β-CDs (grey) and HP-β-CDs (black) in galega water with complexed hawthorn oil.
**Figure 7****.** Fibroblast viability measurements with trypan blue and hemocytometer at 24, 48 and 72 hours. The MIX complex significantly induces fibroblast proliferation.
**Figure 8****.** Numerical results of fibroblast proliferation with trypan blue and hemocytometer at 24, 48 and 72 hours. Two-way ANOVA statistical significance was used with respect to the control, resulting in Mix with a p=0.0032, highly significant.
**Figure 9****.** MMT survival: Mix doesn't induce cellular toxicity at 24 and 48 hours (upper images) and doesn't induce changes in the morphology of fibroblasts (lower images). Live-dead test (life technologies). Simultaneous display of live (green) and dead (red) cells.
**Figure 10****.** Antioxidant activity. Decrease in the concentration of metalloproteinases 1 (MPP1) compared to control through Human MMP-1 ELISA Kit; RAB0361-1KT and its comparison with other active ingredients such as SCA^{®} Growth Factor Technology, algae (algae) and DC (*Senecio rufescens* extract).
**Figure 11****.** A slight increase in the IHC (protein expression) fibroblast activity of Actin (upper image) and Vinculin (Lower image) is observed in the medium with Mix.
**Figure 12****.** Mix influences by inducing the level of FAK (Focal adhesion kinase) and Tubulin proteins.
**Figure 13****.** Regenerative/reparative effect on cellular senescence induced by hydrogen peroxide. Regenerative/reparative effect on mTOR kinase and inhibition of senescent activity of hydrogen peroxide.
**Figure 14****.** Regenerative/reparative effect on cellular senescence induced by hydrogen peroxide. Regenerative/reparative effect on Beta-galactosidase (β-GAL) and inhibition of senescent activity of hydrogen peroxide.
**Figure 15****.** Regenerative/reparative effect on cellular senescence induced by hydrogen peroxide. Regenerative/reparative effect on gen SIR-1(Sirtuine-1) and AGEs (advanced glycation end-products) and inhibition of senescent activity of hydrogen peroxide.
**Figure 16****.** Regenerative/reparative effect on cellular senescence induced by hydrogen peroxide. Regenerative/reparative effect on procollagen and inhibition of senescent activity of hydrogen peroxide. The redensifying effect after induced senescence is milder.
**Figure 17****.** IHC (protein expression) of keratinocytes activity of Vinculin (upper image) and E-Cadherine (Lower image) is observed in the medium with Mix. Mix could have a certain clinical impact on the elasticity of the epidermis.
**Figure 18****.** IHC (protein expression) of keratinocytes activity of β-Catenin is observed in the medium with Mix, although the differences at the level of β-Catenin are slight.

### Detailed description of the invention

The present invention is illustrated by means of the Examples set below without the intention of limiting its scope of protection.

### Example 1. Materials and methods

The inventors performed solubility studies in an aqueous media in the absence and presence of solvents and in the presence of increasing concentrations of different types of CDs (β-CDs and HP-β-CDs).

The addition of cyclodextrins is a formulation strategy often used to increase the apparent aqueous solubility of poorly soluble drugs. The formation of water-soluble inclusion complexes between the drug and cyclodextrin increases the amount of drug in solution, but other mechanisms may also contribute to the increase in drug solubility.

A*_{L}*-type phase solubility (PS) diagrams are characterized by an initial linear increase (AL) in the amount of dissolved drug followed by a plateau in which the concentration of dissolved drug is independent of the concentration of CD. They occur due to the formation of complexes with limited aqueous solubility, but more recent studies suggest that the formation of nanometer- and micrometer-sized aggregates also plays an important role.

For the natural β- and γ CDs, BS-type PS diagrams have been reported which are characterized by an initial linear increase in the amount of dissolved drug followed by a plateau where the concentration of dissolved drug is independent of the CD concentration.

After the plateau, the drug concentration decreases with increasing amounts of CD. From a formulation point of view, Bs-type systems are problematic as just the right amount of CD is required to obtain maximum drug solubility and addition of too much CD (relative to the amount of drug) may significantly lower the amount of dissolved drug.

According to the traditional interpretation, this study was carried out using the solubility method. The reaction medium contained an excess of sea buckthorn oil and resveratrol and different concentrations of CDs in galega water. The samples were sonicated for 60 minutes at 25°C, until an equilibrium was reached, and were subsequently filtered and titrated using a spectrophotometer at 80% ethanol, at the maximum absorbance of sea buckthorn oil (390 nm) and resveratrol (312 nm).

The concentration of resveratrol was calculated using its molar extinction coefficient (28.994 M⁻¹·cm⁻¹) and the Beer-Lambert law: Absorbance = 1·C·ε, where l is the optical cuvette pitch (1 cm), C is the concentration (M) and ε is the molar extinction coefficient (M⁻¹·cm⁻¹).

On the other hand, further sea buckthorn oil solubilization tests were performed with galega water solutions containing 80 mM HP-β-cyclodextrin and another solution with 10 mM β-cyclodextrin.

### Example 2. Results

### Example 2.1. Solubility study

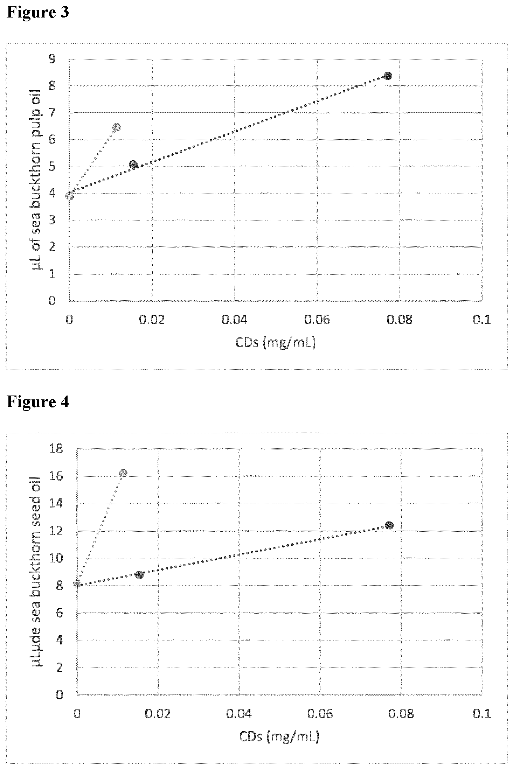

Firstly, the solubility study of pulp and seed sea buckthorn oil in β- and HP-β-CDs was performed. The β-CDs proved to be more effective in the complexation of sea buckthorn oil than their modified HP-β-CDs (**Figures 3 and 4**) obtaining in both cases twice the solubility than in the absence of CDs.

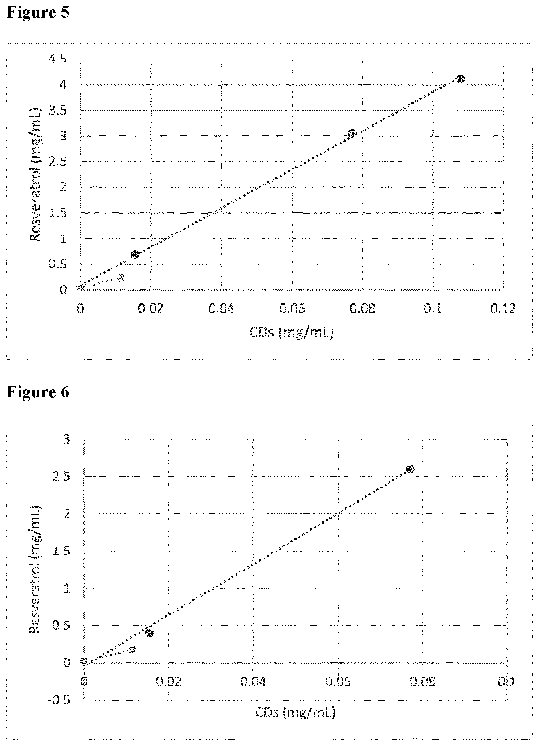

Subsequently, the complexation of resveratrol with β- and HP-β-CDs was studied, and it was observed that modified CDs solubilized more resveratrol, achieving 100 times more soluble resveratrol with HP-β-CDs than without CDs (**Figure 5** **and Table 1**) while with β-CDs the inventors managed to solubilize resveratrol almost 6 times more than without CDs (**Figure 5** **and Table 1**).

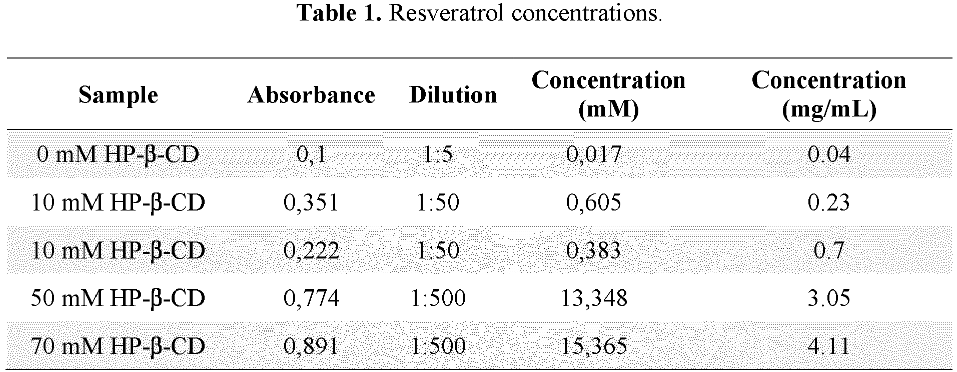

Having studied the complexation of sea buckthorn oil and resveratrol separately, the inventors went on to study the complexation of sea buckthorn oil together with resveratrol. To do this they first complexed sea buckthorn oil in CDs (since it complexes less than resveratrol). In a second stage the inventors added resveratrol to galega water with β-CDs and HP-β-CDs and previously complexed sea buckthorn oil.

As can be seen in **Figure 6** they obtained a high complexation of resveratrol together with sea buckthorn oil (without finding significant differences between pulp and seed sea buckthorn oil). In this case it increases its solubility in the presence of HP-β-CDs 100 times (up to 2.5 mg/mL) than in their absence (0.025 mg/mL). And in the case of β-CDs the solubility of resveratrol is increased 7 times (0.18 mg/mL) than in the absence of them (0.025 mg/mL).

For the scientific studies, a solution of Resveratrol and sea buckthorn pulp (*Hippophae Rhamnoides*) complexed in beta dextrins was used on a solution of *Galega Officinalis* with the following proportions:
- Solution of Reveratrol cyclodextrinated to remain at 2,5 mg/mL
- Sea buckthorn cyclodrextrinated at a concentration of 8 µL/mL

This composition is hereinafter referred to as "the active Mix", "the Mix" or "the MIX".

### Example 2.2. Control of ROS

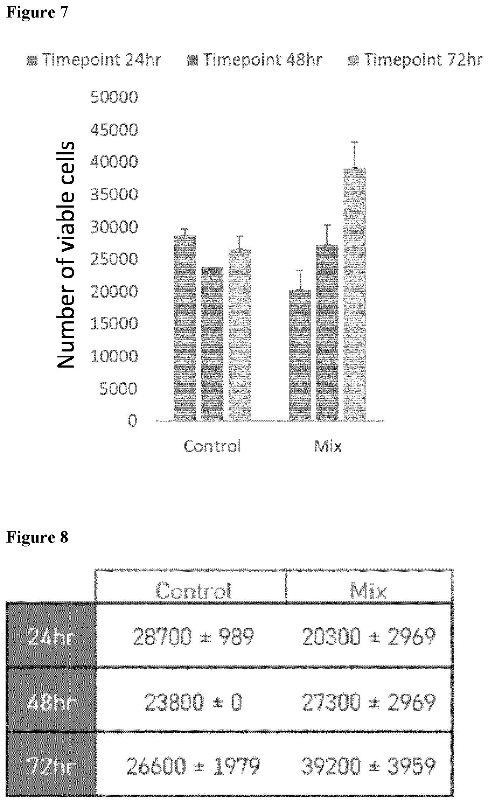

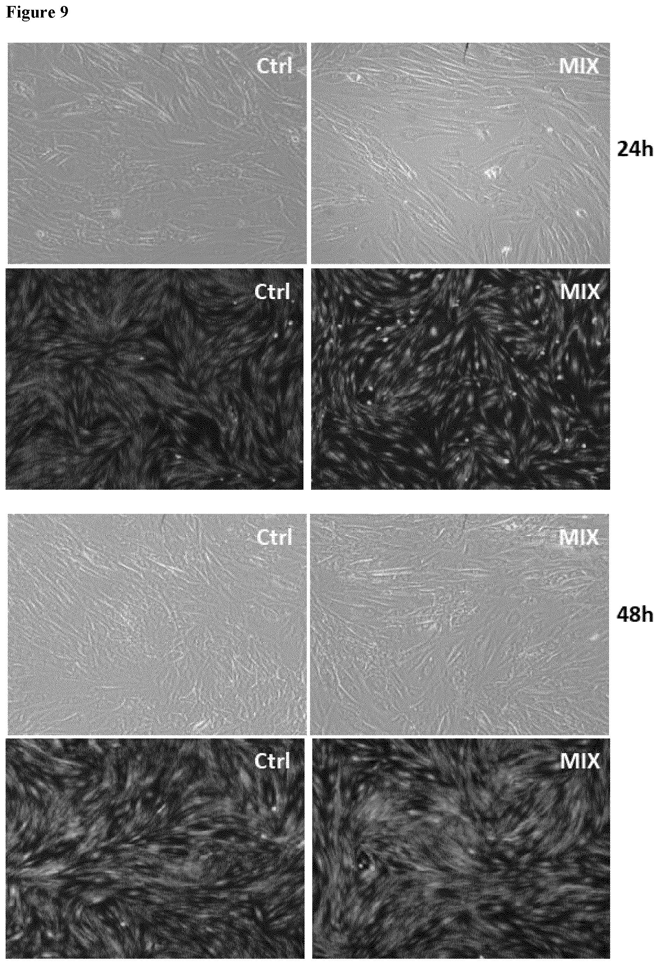

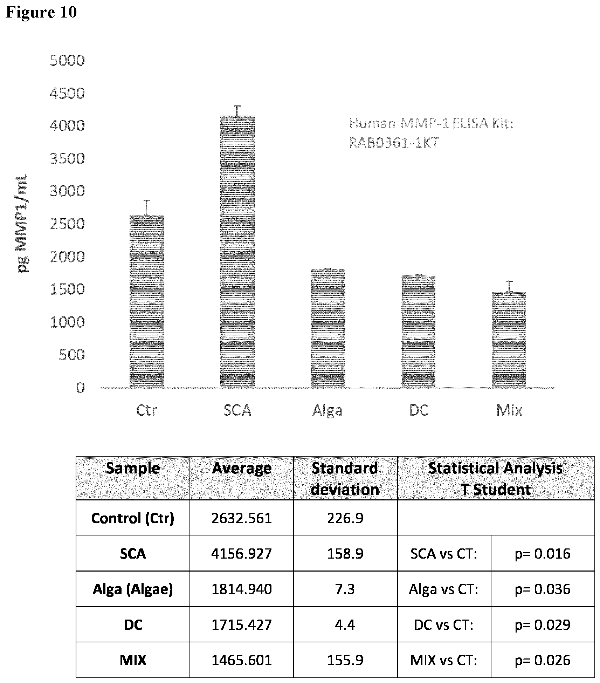

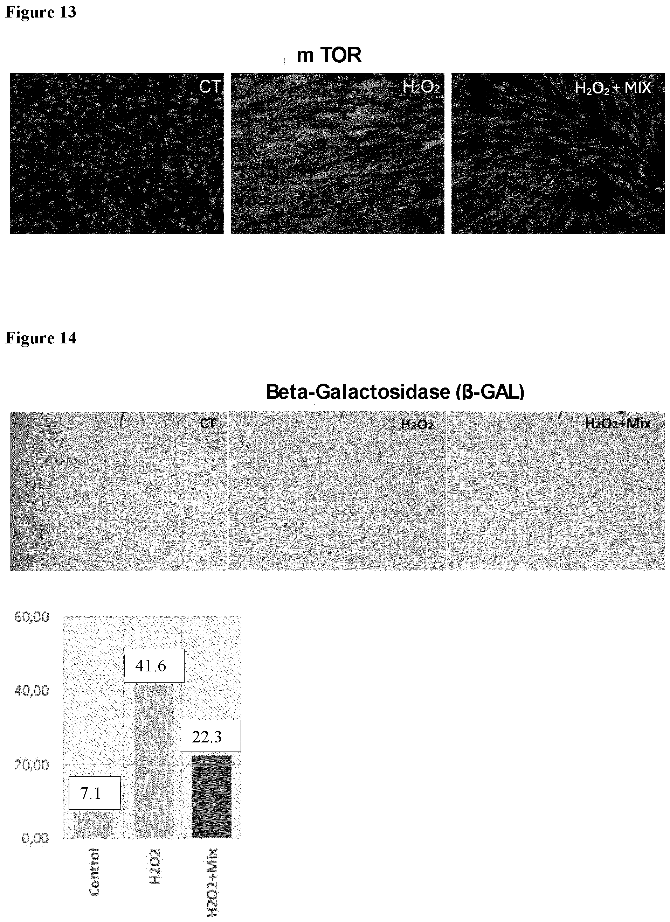

Various tests were carried out on cell cultures of fibroblasts and keratinocytes of the active Mix to establish viability or its influence on the different growth factors and molecules that determine cell viability.
- Cell survival and proliferation were studied on fibroblast cell cultures (**Figures 7** and **8**), as well as the cellular toxicity of the MIX preparation (**Figure 9**). The decrease in metalloproteinases and its consequent antioxidant protective capacity on cells was assessed through ELISA (**Figure 10**), comparing it with other active ingredients that have demonstrated the ability to exert this activity.
- A second part of the study was dedicated to determining the activity of the MIX complex against the prevention of senescence caused in fibroblast culture by the addition of hydrogen peroxide (H2O2).
   The reparative effect on senescence induced with H2O2 on β-Gal (Beta-galactosidase), mTOR, SIRT1, AGEs and Procollagen was determined. (**Figures 13, 14****,** **15 and 16**)
- Finally, the effect of MIX on keratinocytes and its influence on epidermal elasticity determined through Vinculin, E-cadherin and β-Catenin was studied.

### Example 2.3. Proliferation, morphology, movement, activity, and toxicity on fibroblasts

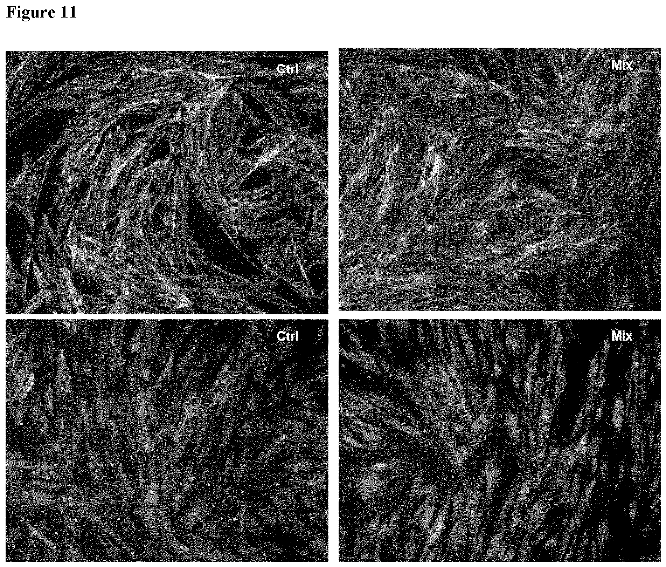

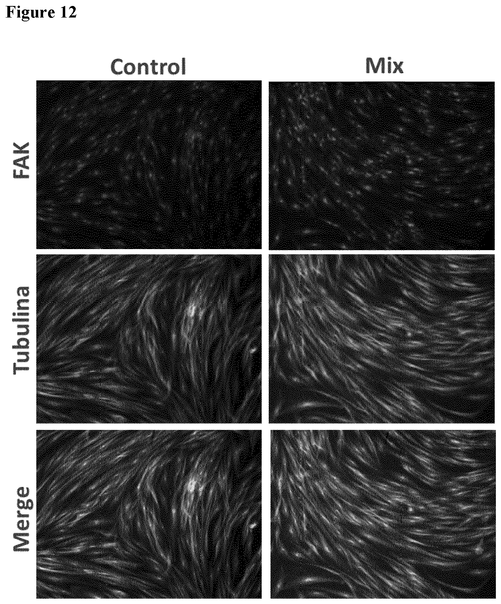

- Increases proliferation and survival of fibroblasts (Renovator effect).
   As can be seen in **Figure 7**, the number of fibroblasts increases progressively after 24 hours when the MIX mixture is added, especially after 72 hours of life of the cell culture. This determination is statistically significant (p=0.0032) compared to the control and goes from 26,600 ± 1979 to 39,200 ± 3959 viable cells, that is, the proliferation of fibroblasts increases by 147% (**Figure 8**). Acts on fibronectin: Secretion, synthesis and assembly (Redensifying). Its main function in our body is to firm the skin and make the upper layer or epithelial layer firm and strong. An important function of fibronectin at the level of connective tissue is the formation of fibrils in areas of tissue remodelling such as it occurs in the healing of an injury, where a provisional matrix is required for cellular union and dissemination.
   Otherwise, cell survival (MTT) is not affected (**Figure 9**). The MTT assay is used to measure cellular metabolic activity as an indicator of cellular viability, proliferation, and cytotoxicity.
- Decreases MPP1 and MPP2 (Redensifying). Metalloproteinases are endopeptidases that perform a degradative function, generally targeting the extracellular matrix of cells. The antioxidant capacity of a substance can be determined by the decrease in the concentration of MMP. This property can be determined through an ELISA test and as was our case, it was compared with other substances with recognized antioxidant capacity. The result indicated that the greatest decrease in MMP occurred when the MIX preparation was in the cell medium, and its statistical significance obtained through paired data of the Student's T obtained a p=0.026 was well below the limit of significance. of p<0.050 (**Figure 10**)
- Slightly increases actin and vinculin (Redensifying) Actin is a filamentous protein of the cell protoplasm, which is responsible for conferring its shape to the cell, fixing membrane proteins, participating in cell movements, and associated with myosin, causing contraction of muscle cells. Vinculin is a membrane-cytoskeleton protein involved in anchoring integrin molecules to the actin cytoskeleton. Integrins are transmembrane glycoproteins involved in cell-to-cell and cell-to-extracellular matrix adhesion. Our studies allow us to conclude a slight increase in the IHC (protein expression) fibroblast activity of Actin (upper image) and Vinculin (Lower image) is observed in the medium with MIX (**Figure 11**).
- Induces at the level of FAK/P-FAK protein and tubulins (Redensifying). Focal adhesion kinase (FAK), phosphorylated or not, is an anchor protein responsible for keeping the cell attached to the extracellular matrix, as well as maintaining cell-cell contact. FAK is involved in multiple cell survival mechanisms such as proliferation, motility and migration. Tubulins are the fundamental protein elements of microtubules (narrow, hollow tubes inside a cell), which are involved in cell multiplication and movement. Our assays allow us to affirm that the MIX complex induces the formation of FAK and Tubulin proteins (**Figure 12**).

### Example 2.4. Control of senescence (aged fibroblasts)

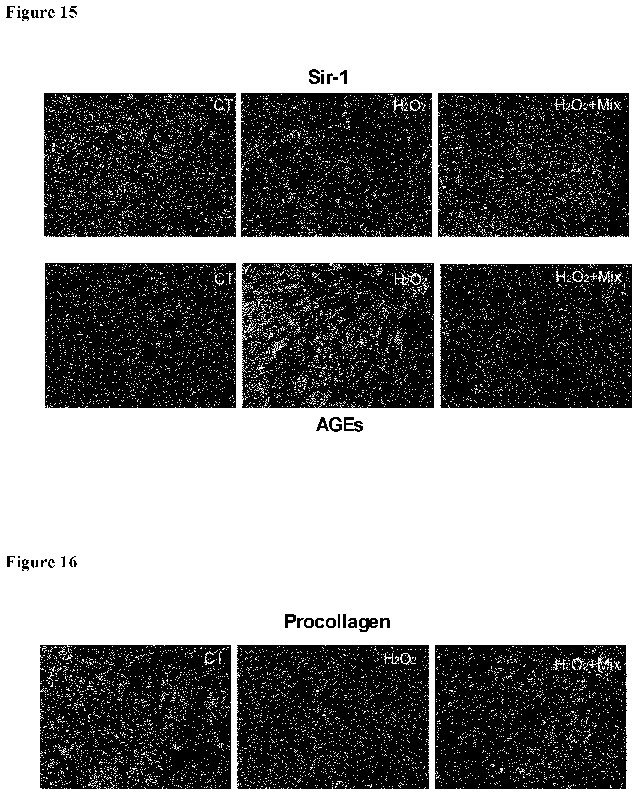

Several tests were carried out with the MIX preparation in order to validate its antioxidant inhibitory effect and cell viability, after addition of hydrogen peroxide to the fibroblast culture medium in order to provoke cellular senescence in it and determine a repairing effect on senescence induced. with H2O2 on mTOR, β-Gal (Beta-galactosidase), SIRT1, AGEs (Renovator) and Procollagen (Redensifier).
- mTOR is the key to cellular catabolism and anabolism that determines whether cells, and in particular cancer cells, should grow and proliferate. In addition, mTOR has effects on the regulation of apoptosis. In our experience, the MIX complex has shown that it partially inhibits the senescent effect caused by the incorporation of oxygenated water into the cellular medium (**Figure 13**).
- Galactosidase is an enzyme that catalyses the hydrolysis of galactosides to monosaccharides in the cellular enzymatic process. In humans, β-galactosidase cleaves lactose so it can be absorbed as galactose and glucose essential for cellular life whose effectiveness declines in senescence. Our assays demonstrate an effectiveness close to 50% in inhibiting senescence caused by the addition of hydrogen peroxide to the cellular medium (**Figure 14**).
- SIRT1 is a protein that has important functions in the cell. Among these functions are DNA damage repair and resistance to cellular and metabolic stress. It also participates in the lengthening of telomeres and protects the genome during the cellular reprogramming process. SIRT1 activity is linked to aging and regulation of intake. Sirtuins are a family of NAD-dependent deacetylases that play a fundamental role in a large number of biological processes such as the regulation of the energy state of the cell, cell differentiation and aging. The MIX complex has demonstrated a regenerative-reparative effect on senescence caused by the addition of oxygenated water to the fibroblast cell medium (**Figure 15**).

- AGEs (advanced glycation end-products, AGE) are modified proteins that appear at the tissue and plasma level as a result of the reaction of monosaccharides present in the blood with the basic amino acids of proteins. They are responsible for cellular aging and act by various mechanisms, including glycation of biomolecules. The MIX complex has demonstrated a clear regenerative-reparative effect on senescence caused by the addition of oxygenated water to the fibroblast cell medium (**Figure 15**).
- Procollagen is a protein that the organism produces in skin, hair, tendons, cartilage, bones and joints from birth until approximately 20 years of age. Thereafter, production begins to decline and by the age of 60 the body produces less than 70% of the procollagen needed for healthy, youthful and revitalized skin. The MIX complex has demonstrated a regenerative-reparative effect on senescence caused by the addition of oxygenated water to the fibroblast cell medium (**Figure 16**).

### Example 2.5. Effect on keratinocytes

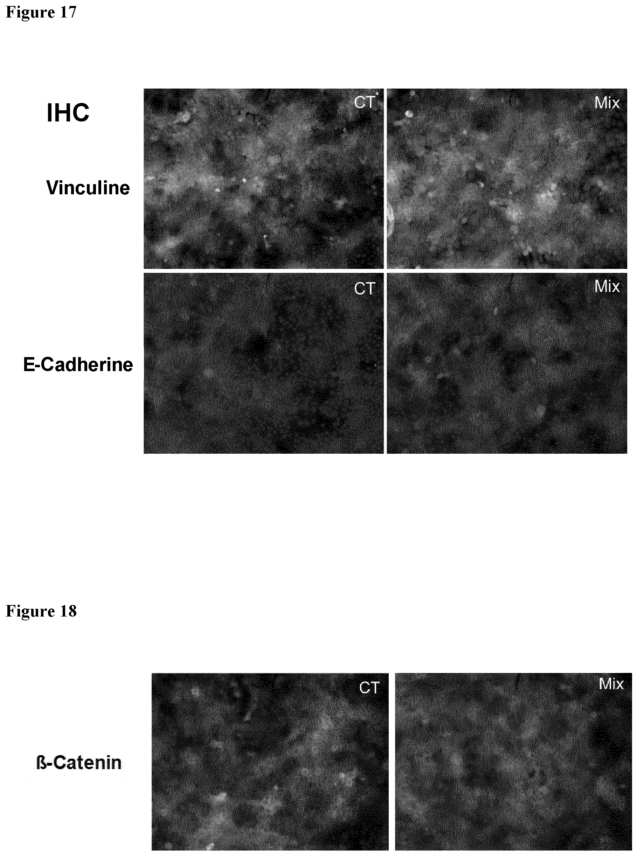

Finally, we studied the possible effects that the MIX complex could have on the regeneration, elasticity and adhesion of the epidermis and specifically its influence on the viability of the stratum corneum improves the elasticity of the epidermis through Vinculin, E-cadherin and β-Catenin (Redensifying).
- Vinculin is in the adhesion of the cytoskeleton to the adhesion transmembrane molecules. Cell diffusion and movement occur through the process of binding between the integrin receptors on the cell surface and the adhesion molecules of the extracellular matrix. Vinculin is associated with focal adhesions and adherens junctions. It has been postulated that the lack of vinculin could decrease cell adhesion by inhibiting the assembly of focal adhesions and preventing actin polymerization. The MIX complex can influence proper epidermal formation and favorably influence its elasticity (**Figure 17**).
- E-cadherin is a transmembrane glycoprotein involved in cell adhesion in epithelia. It is necessary so that diverse cells can migrate as a group. This protein is a type of adhesive that keeps cells tightly together, promoting the organization of tissues and organs. These molecules are transmembrane glycoproteins responsible for cell-cell junctions to maintain epidermal integrity. The MIX complex can influence proper epidermal formation and favorably influence its elasticity (**Figure 17**).
- β-catenin regulates processes such as tissue regeneration, stem cell differentiation and cell proliferation. It participates in the formation of adherens junctions of mammalian epithelia, helping to anchor cadherins to the actin cytoskeleton. The MIX complex can influence proper epidermal formation and favorably influence its elasticity (**Figure 17**).

### Example 2.5. Antioxidant activity

Trolox equivalent antioxidant capacity (TEAC) is a measure of antioxidant strength based on its comparison to Trolox (6-hydroxy-2,5,7,8-tetramethylchroman-2-carboxylic acid), a vitamin E analogue, which is soluble in water and measures antioxidant power in units called Trolox equivalents (TE).

Due to the difficulties in measuring individual antioxidant components of a complex mixture, Trolox Equivalence is used as a reference for the antioxidant capacity of such a mixture. Trolox equivalence is most frequently measured using the ABTS decolorization assay. The TEAC assay is used to measure the antioxidant capacity of foods, beverages and supplements.

As shown in **Table 2**, the antioxidant activity of the mix is unexpectedly higher than that of galega water, sea buckthorn extract and resveratrol alone. Of note, resveratrol 0.25% is the maximum solubility of resveratrol in water, and therefore it is a higher concentration of resveratrol than the one used in the final mix. Even under these circumstances, the final mix presented an improved antioxidant concentration than resveratrol alone, which further emphasizes that the antioxidant activity of the mix is unexpectedly higher than that of galega water, sea buckthorn extract and resveratrol alone. These results reveal a synergistic interaction between the compounds.

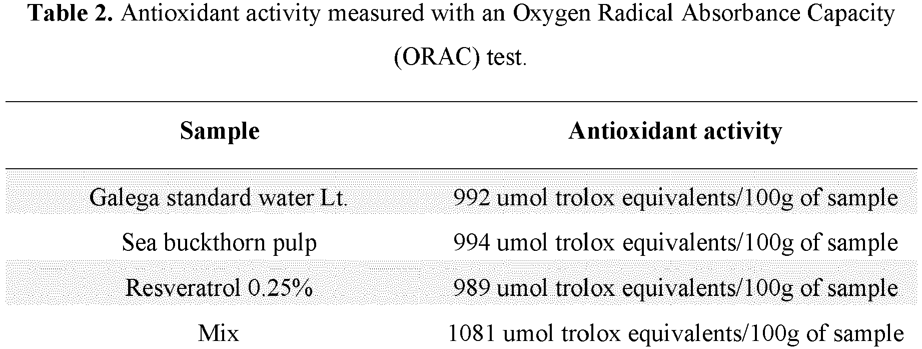

## Claims

1. Method for obtaining a composition comprising crude water extract of *Galega officinalis L.* water, *Hippophae rhamnoides* oil and resveratrol, comprising the steps of:
a. solubilizing *Hippophae rhamnoides* oil in crude water extract of *Galega officinalis L.* through its molecular encapsulation with complexing agents,
b. solubilizing resveratrol in crude water extract of *Galega officinalis L.* through its molecular encapsulation with complexing agents, and
c. mixing both preparations and filtering the mixture.

2. Method, according to claim 1, **characterized in that** the complexing agent of step a) is β-cyclodextrin, or **in that** the complexing agent of step b) is 2-hydroxypropyl-β-cyclodextrin.

3. Method, according to claim 2, **characterized in that** the complexing agent of step a) is β-cyclodextrin, and **in that** the complexing agent of step b) is 2-hydroxypropyl-β-cyclodextrin.

4. Anti-ageing composition obtainable by any of the methods of claims 1 to 3.

5. Anti-ageing composition, according to claim 4, **characterized in that** the *Hippophae rhamnoides* oil is extracted from the pulp and/or from the seed of the plant.

6. Anti-ageing composition, according to any of the claims 4 to 5, wherein the crude water extract of *Galega officinalis L.* comprises biguanides.

7. Anti-ageing composition, according to any of the claims 4 to 6, **characterized in that** the resveratrol is at a final concentration of 10.0 mg/mL to 1.0 mg/mL, preferably at a final concentration of 2.5 mg/mL.

8. Anti-ageing composition, according to any of the claims 4 to 7, **characterized in that** the *Hippophae rhamnoides* oil is at a final concentration of 25 µL/mL to 1 µL/mL, preferably at a final concentration of 8 µL/mL.

9. Anti-ageing composition, according to any of the claims 4 to 8, for use as a medicament.

10. Anti-ageing composition for use, according to claim 9, in a method for the treatment of skin aging, the prevention of skin ageing, the promotion of skin redensification, the reduction of fibroblast senescence, the reduction of metalloproteinase function, the promotion of skin regeneration and/or the promotion of cutaneous healing.

11. Cosmetic use of the composition of any one of claims 4 to 8 for skin care or maintenance, preferably for decreasing a sign of cutaneous aging, for renovating the skin, for regenerating the skin, for preventing or treating skin desquamation, for skin depigmentation, for reducing skin inflammation and/or for promoting cutaneous healing, more preferably for decreasing a sign of cutaneous aging.

12. Cosmetic method for skin care or maintenance, comprising applying or administering to a subject a preparation for skin care or maintenance, wherein the preparation comprises the composition of any one of claims 4 to 8

13. Cosmetic method, according to claim 12, wherein the subject is interested in achieving a decrease in a sign of skin aging.

14. Anti-ageing composition for use according to claims 9 or 10, cosmetic use according to claim 11, or cosmetic method according to claims 12 or 13 wherein the composition is administered topically.

15. Anti-ageing composition for use according to claims 9, 10 or 14, or cosmetic method according to claim 12, 13 or 14, wherein the method comprises obtaining a composition comprising crude water extract of *Galega officinalis L.* water, *Hippophae rhamnoides* oil and resveratrol using the any of the methods of claims 1 or 2.
